# EUROPEAN PATENT APPLICATION

(11) **EP 1 495 766 A1**
(43) Date of publication of application: **12.01.2005**
(21) Application number: 03015528.7
(22) Date of filing: 10.07.2003
(51) Int. Cl.: A61K 38/48, A61P 35/00, A61P 17/06

(54) **Factor VII activating protease (FSAP) derived polypeptides for the treatment of angiogenesis-related disorders**

(71) Applicant: ZLB Behring GmbH, 35041 Marburg (DE)
(72) Inventor: Weimer, Thomas, Gladenbach (DE); Celik, Ilhan, 35085 Ebsdorfergrund (DE); Kanse, Sandip, 35440 Linden-Leihgestern (DE)

(57) **Abstract**

The invention relates to the use of the coagulation factor VII activating protease, fragments and variants thereof in the prevention, treatment or amelioration of angiogenesis-related diseases or disorders.

## Description

The invention relates to the use of the coagulation factor VII activating protease, fragments and variants thereof in the prevention, treatment or amelioration of angiogenesis-related diseases or disorders.

The German patent application 199 03 693.4 discloses a protease isolated from blood plasma, that is capable of activating the coagulation factor VII. Also previously described therein is a process for obtaining it, detecting it and inactivating it, and pharmaceutical preparations containing this protease. In accordance with its properties, this protease is referred to as factor seven activating protease (=FSAP). The German patent application 199 03 693.4 additionally reports that FSAP has a plasminogen activator activating and/or potentiating action which can be measured through the activation of single-chain urokinase plasminogen activator or single-chain tissue plasminogen activator.

The German patent application 199 03 693.4 has also described processes and test systems for the qualitative and quantitative detection of FSAP that are based on the measurement of a reduction in blood clotting times and the activation of plasminogen activators. Test systems of these types also allow FSAP to be measured in complex protein solutions such as plasma, as described in particular in the German patent application 199 26 531.3.

It has additionally been disclosed that FSAP influences hemostasis and the cellular processes connected therewith. Through it's involvement in blood clotting and/or fibrinolysis it also acts on the wound-healing response. Other previously disclosed characteristics are the strong binding to hyaluronic acid and glycosaminoglycans and the rapid autoactivation of the proenzyme in the presence of, for example, heparin or dextran sulfate. The property of binding to negatively charged surfaces, and the high affinity for heparin that the protease interacts with cell surfaces and extracellular matrix. Patent application DE 101 48 037.7, the contents of which are hereby expressly included in this application, describes a concentration-dependant reduction of smooth muscle cell proliferation by FSAP.

It has now been found that proliferation and migration of growth-factor stimulated endothelial cells can be significantly inhibited by FSAP. Inhibition of FSAP by, for example, aprotinin led to a reduction in this effect. Proliferation and migration of endothelial cells is a prerequisite for angiogenesis. In an in vivo model for angiogenesis, application of FSAP resulted in significant reduction of blood-vessel immigration into a growth-factor containing matrigel implant. Furthermore, in a mouse tumor model, FSAP led to an about 50% inhibition of pancreatic tumor cell transplant growth in SCID mice, demonstrating an anti-angiogenic effect of FSAP in vivo.

The invention therefore relates to the use of FSAP for the prophylaxis, therapy or amelioration of angiogenesis-related disorders as specified below. In the present invention, the term FSAP is collectively used for the factor VII activating protease molecule of human and other species, its proenzymes, fragments, variants (including mutants) and analogues thereof which display angiogenesis inhibiting properties. It is understood by the one skilled in the art that the proteins or peptides can be of natural, synthetic or recombinant origin. Constructs encoding FSAP proteins of the invention can be used as a part of a gene therapy protocol to deliver therapeutically effective doses of the FSAP protein.

The naturally occurring balance between endogenous stimulators and inhibitors of angiogenesis is one in which inhibitory influences predominate. Carmeliet, Nature Medicine 9:653-660 (2003); Hanahan D, Folkman J. Patterns and emerging mechanisms of the angiogenic switch during tumorigenesis. Cell 1996; 86(3):353-364; Hanahan D, Weinberg RA. The hallmarks of cancer. Cell 2000; 100(1):57-70; Kerbel R, Folkman J. Clinical translation of angiogenesis inhibitors. Nat Rev Cancer 2002; 2(10):727-739. In such conditions in which neovascularization occurs under normal physiological conditions, such as wound healing, organ regeneration, embryonic development, and female reproductive processes, angiogenesis is tightly regulated and spatially and temporally delimited. Under conditions of pathological angiogenesis such as that characterizing solid tumor growth, these regulatory controls fail. Unregulated angiogenesis becomes pathologic and sustains progression of many neoplastic and non-neoplastic diseases. A number of serious diseases are dominated by abnormal neovascularization including solid tumor growth and metastases, arthritis, some types of eye disorders, inflammatory bowel disease and psoriasis. See, e.g., reviews by Hanahan D, Folkman J. Patterns and emerging mechanisms of the angiogenic switch during tumorigenesis. Cell 1996; 86(3):353-364; Carmeliet P, Jain RK. Angiogenesis in cancer and other diseases. Nature 2000; 407(6801):249-257; Hanahan D, Weinberg RA. The hallmarks of cancer. Cell 2000; 100(1):57-70; Folkman J. Tumor angiogenesis. In: Holland JF, Frei E, Bast RCJr, Kufe DW, Pollock RE, Weichselbaum RR, editors. Cancer Medicine. Ontario, Canada: B.C. Decker Inc., 2000: 132-152; Folkman J. Angiogenesis-dependent diseases. Semin Oncol 2001; 28(6):536-542; Folkman J. Angiogenesis. In: Braunwald E, Fauci AS, Kasper DL, Hauser SL, Longo DL, Jameson JL, editors. Harrison's textbook of internal medicine. McGraw-Hill, 2001: 517-530; Jain RK. Tumor angiogenesis and accessibility: role of vascular endothelial growth factor. Semin Oncol 2002; 29(6 Suppl 16):3-9; Kerbel R, Folkman J. Clinical translation of angiogenesis inhibitors. Nat Rev Cancer 2002; 2(10):727-739; Folkman J. Role of angiogenesis in tumor growth and metastasis. Semin Oncol 2002; 29(6 Suppl 16):15-18; Folkman J. Angiogenesis and apoptosis. Semin Cancer Biol 2003; 13(2):159-167. In a number of pathological conditions, the process of angiogenesis contributes to the disease state. For example, significant data have accumulated which suggest that the growth of solid tumors is dependent on angiogenesis. Folkman and Klagsbrun, *Science 235*:442-447 (1987); Kerbel R, Folkman J. Clinical translation of angiogenesis inhibitors. Nat Rev Cancer 2002; 2(10):727-739. Angiogenesis in this context includes, but is not limited to, other related processes such as arteriogenesis, vasculogenesis, collateral growth and growth of new vessels from endothelial progenitor cells.
The present invention provides for treatment of diseases or disorders associated with neovascularization by administration of FSAP proteins of the invention and/or polynucleotides encoding FSAP proteins of the invention. Malignant and metastatic conditions which can be treated with the polynucleotides and polypeptides, or agonists or antagonists of the invention include, but are not limited to, malignancies, solid tumors, and cancers described herein and otherwise known in the art (for a review see Carmeliet, Nature Medicine 9:653-660 (2003)). Thus, the present invention provides a method of treating an angiogenesis-related disease and/or disorder, comprising administering to an individual in need thereof a therapeutically effective amount of a FSAP protein of the invention and/or polynucleotides encoding a FSAP protein of the invention. For example, FSAP proteins of the invention and/or polynucleotides encoding FSAP proteins of the invention may be utilized in a variety of additional methods in order to therapeutically treat a cancer or tumor. Cancers which may be treated with FSAP proteins of the invention and/or polynucleotides encoding FSAP proteins of the invention include, but are not limited to solid tumors, including prostate, lung, breast, ovarian, stomach, pancreas, larynx, esophagus, testes, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non- small cell lung cancer; colorectal cancer; advanced malignancies; and blood born tumors such as leukemias.

The route of administration includes, but is not limited to, intravenous (i.v.), subcutaneous (s.c.), intradermal, intra peritoneal (i.p.; within the abdominal cavity), oral (p.o.), intravesical (instillation into the bladder); intrathecal (instillation into cerebral liquor), intra-uterine (within the uterus), intra-articular. Sponges, mesh and suture material loaded with FSAP.

Administration of FSAP as a spray; aerosol, ointment (unguentum), cream, drop, tincture. Furthermore FSAP could be administrated by bolus injection or continuous administration by pump (e.g. i.v.; s.c.; i.p etc.). Treatment with FSAP could be combined with chemotherapy, radiation or both, immunotherapy and gene therapy. It can be used for patients with advanced tumors (metastasis etc.) and non-advanced tumors. It can be used in combination with other antiangiogenic substances or therapies. For example, FSAP proteins of the invention and/or polynucleotides encoding FSAP proteins of the invention may be delivered topically, in order to treat cancers such as skin cancer, head and neck tumors, breast tumors, and Kaposi's sarcoma.

Within yet other aspects, FSAP proteins of the invention and/or polynucleotides encoding FSAP proteins of the invention may be utilized to treat superficial forms of bladder cancer by, for example, intravesical administration. FSAP proteins of the invention and/or polynucleotides encoding FSAP proteins of the invention may be delivered directly into the tumor, or near the tumor site, via injection or a catheter. Of course, as the artisan of ordinary skill will appreciate, the appropriate mode of administration will vary according to the cancer to be treated. Other modes of delivery are discussed herein.

FSAP proteins of the invention and/or polynucleotides encoding FSAP proteins of the invention may be useful in treating other disorders, besides cancers, which involve angiogenesis. These disorders include, but are not limited to: benign tumors, for example hemangiomas, acoustic neuromas, neurofibromas, trachomas, and pyogenic granulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygia (abnormal blood vessel growth) of the eye; rheumatoid arthritis; psoriasis; delayed wound healing; endometriosis; vasculogenesis; granulations; hypertrophic scars (keloids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroma; fibromuscular dysplasia; wound granulation; Crohn's disease; and atherosclerosis. For an extensive list of such conditions see Carmeliet, Nature Medicine 9:653-660 (2003), which list is hereby incorporated by reference.

For example, within one aspect of the present invention methods are provided for treating hypertrophic scars and keloids, comprising the step of administering FSAP proteins of the invention and/or polynucleotides encoding FSAP proteins of the invention to a hypertrophic scar or keloid. Within one embodiment of the present invention FSAP proteins of the invention and/or polynucleotides encoding FSAP proteins of the invention are directly injected into a hypertrophic scar or keloid, in order to prevent the progression of these lesions. This therapy is of particular value in the prophylactic treatment of conditions which are known to result in the development of hypertrophic scars and keloids (e.g., burns), and is optionally initiated after the proliferative phase has had time to progress (approximately 14 days after the initial injury), but before hypertrophic scar or keloid development. As noted above, the present invention also provides methods for treating neovascular diseases of the eye, including for example, corneal neovascularization, neovascular glaucoma, proliferative diabetic retinopathy, retrolental fibroplasia and macular degeneration.

Moreover, Ocular disorders associated with neovascularization which can be treated with the FSAP proteins of the invention and/or polynucleotides encoding FSAP proteins of the invention include, but are not limited to: neovascular glaucoma, diabetic retinopathy, retinoblastoma, retrolental fibroplasia, uveitis, retinopathy of prematurity macular degeneration, corneal graft neovascularization, as well as other eye inflammatory diseases, ocular tumors and diseases associated with choroidal or iris neovascularization. See, e.g., reviews by Waltman *et al.*, *Am. J. Ophthal. 85*:704-710 (1978) and Gartner *et al.*, *Surv. Ophthal. 22*:291-312 (1978).

Thus, within one aspect of the present invention methods are provided for treating neovascular diseases of the eye such as corneal neovascularization (including corneal graft neovascularization), comprising the step of administering to a patient a therapeutically effective amount of a compound (e.g., FSAP proteins of the invention and/or polynucleotides encoding FSAP proteins of the invention) to the cornea, such that the formation of blood vessels is inhibited. Briefly, the cornea is a tissue which normally lacks blood vessels. In certain pathological conditions however, capillaries may extend into the cornea from the pericorneal vascular plexus of the limbus. When the cornea becomes vascularized, it also becomes clouded, resulting in a decline in the patient's visual acuity. Visual loss may become complete if the cornea completely opacitates. A wide variety of disorders can result in corneal neovascularization, including for example, corneal infections (e.g., trachoma, herpes simplex keratitis, leishmaniasis and onchocerciasis), immunological processes (e.g., graft rejection and Stevens-Johnson's syndrome), alkali burns, trauma, inflammation (of any cause), toxic and nutritional deficiency states, and as a complication of wearing contact lenses.

Within yet further embodiments of the invention, FSAP proteins may be prepared for topical administration in saline (combined with any of the preservatives and antimicrobial agents commonly used in ocular preparations), and administered in eyedrop form. The solution or suspension may be prepared in its pure form and administered several times daily. Alternatively, anti-angiogenic compositions, prepared as described above, may also be administered directly to the cornea. Within other embodiments, the anti-angiogenic composition is prepared with a muco-adhesive polymer which binds to cornea. Within further embodiments, the anti-angiogenic factors or anti-angiogenic compositions may be utilized as an adjunct to conventional steroid therapy. Topical therapy may also be useful prophylactically in corneal lesions which are known to have a high probability of inducing an angiogenic response (such as chemical burns). In these instances the treatment, likely in combination with steroids, may be instituted immediately to help prevent subsequent complications.

Within other embodiments, the compounds described above may be injected directly into the corneal stroma by an ophthalmologist under microscopic guidance. The site of injection may vary with the morphology of the individual lesion, but the goal of the administration would be to place the composition at the advancing front of the vasculature (i.e., interspersed between the blood vessels and the normal cornea). In most cases this would involve perilimbic corneal injection to "protect" the cornea from the advancing blood vessels. This method may also be utilized shortly after a corneal insult in order to prophylactically prevent corneal neovascularization. In this situation the material could be injected in the perilimbic cornea interspersed between the corneal lesion and its undesired potential limbic blood supply. Such methods may also be utilized in a similar fashion to prevent capillary invasion of transplanted corneas. In a sustained-release form injections might only be required 2-3 times per year. A steroid could also be added to the injection solution to reduce inflammation resulting from the injection itself.

Within another aspect of the present invention, methods are provided for treating neovascular glaucoma, comprising the step of administering to a patient a therapeutically effective amount of a FSAP protein of the invention and/or polynucleotides encoding a FSAP protein of the invention to the eye, such that the formation of blood vessels is inhibited. In one embodiment, the compound may be administered topically to the eye in order to treat early forms of neovascular glaucoma. Within other embodiments, the compound may be implanted by injection into the region of the anterior chamber angle. Within other embodiments, the compound may also be placed in any location such that the compound is continuously released into the aqueous humor. Within another aspect of the present invention, methods are provided for treating proliferative diabetic retinopathy, comprising the step of administering to a patient a therapeutically effective amount of a FSAP protein of the invention and/or polynucleotides encoding a FSAP protein of the invention to the eyes, such that the formation of blood vessels is inhibited.

Within yet further embodiments of the invention, proliferative diabetic retinopathy may be treated by injection into the aqueous humor or the vitreous, in order to increase the local concentration of the polynucleotide, polypeptide, antagonist and/or agonist in the retina. This treatment could be initiated prior to the acquisition of severe disease requiring photocoagulation.

Within another aspect of the present invention, methods are provided for treating retrolental fibroplasia, comprising the step of administering to a patient a therapeutically effective amount of a FSAP protein of the invention and/or polynucleotides encoding a FSAP protein of the invention to the eye, such that the formation of blood vessels is inhibited. The compound may be administered topically, via intravitreous injection and/or via intraocular implants.

Additionally, disorders which can be treated with FSAP proteins of the invention and/or polynucleotides encoding FSAP proteins of the invention include, but are not limited to, hemangioma, arthritis, psoriasis, angiofibroma, atherosclerotic plaques, delayed wound healing, granulations, hemophilic joints, hypertrophic scars, nonunion fractures, Osler-Weber syndrome, pyogenic granuloma, scleroderma, trachoma, and vascular adhesions.

Moreover, disorders and/or states, which can be treated, prevented, diagnosed, and/or prognosed with the FSAP proteins of the invention and/or polynucleotides encoding FSAP proteins of the invention include, but are not limited to, solid tumors, blood born tumors such as leukemias, multiple myelomas, tumor metastasis, Kaposi's sarcoma, benign tumors, for example hemangiomas, acoustic neuromas, neurofibromas, trachomas, and pyogenic granulomas, rheumatoid arthritis, psoriasis, ocular angiogenic diseases, for example, diabetic retinopathy, retinopathy of prematurity, macular degeneration, corneal graft rejection, neovascular glaucoma, retrolental fibroplasia, rubeosis, retinoblastoma, and uvietis, delayed wound healing, endometriosis, vascluogenesis, granulations, hypertrophic scars (keloids), nonunion fractures, scleroderma, trachoma, vascular adhesions, myocardial angiogenesis, coronary collaterals, cerebral collaterals, arteriovenous malformations, ischemic limb angiogenesis, Osler-Webber Syndrome, plaque neovascularization, telangiectasia, hemophiliac joints, angiofibroma fibromuscular dysplasia, wound granulation, Crohn's disease, atherosclerosis, birth control agent by preventing vascularization required for embryo implantation controlling menstruation, diseases that have angiogenesis as a pathologic consequence such as cat scratch disease (Rochele minalia quintosa), ulcers (Helicobacter pylori), Bartonellosis and bacillary angiomatosis.

In one aspect of the birth control method, an amount of the compound sufficient to block embryo implantation is administered before or after intercourse and fertilization have occurred, thus providing an effective method of birth control, possibly a "morning after" method. FSAP proteins of the invention and/or polynucleotides encoding FSAP proteins of the invention may also be used in controlling menstruation or administered as either a peritoneal lavage fluid or for peritoneal implantation in the treatment of endometriosis.

FSAP proteins of the invention and/or polynucleotides encoding FSAP proteins of the invention may be incorporated into surgical sutures in order to prevent stitch granulomas.

FSAP proteins of the invention and/or polynucleotides encoding FSAP proteins of the invention may be utilized in a wide variety of surgical procedures. For example, within one aspect of the present invention a compositions (in the form of, for example, a spray or film) may be utilized to coat or spray an area prior to removal of a tumor, in order to isolate normal surrounding tissues from malignant tissue, and/or to prevent the spread of disease to surrounding tissues. Within other aspects of the present invention, compositions (e.g., in the form of a spray) may be delivered via endoscopic procedures in order to coat tumors, or inhibit angiogenesis in a desired locale. Within yet other aspects of the present invention, surgical meshes which have been coated with anti- angiogenic compositions of the present invention may be utilized in any procedure wherein a surgical mesh might be utilized. For example, within one embodiment of the invention a surgical mesh loaded with an anti-angiogenic composition may be utilized during abdominal cancer resection surgery (e.g., subsequent to colon resection) in order to provide support to the structure, and to release an amount of the anti-angiogenic factor.

Within further aspects of the present invention, methods are provided for treating tumor excision sites, comprising administering FSAP proteins of the invention and/or polynucleotides encoding FSAP proteins of the invention to the resection margins of a tumor subsequent to excision, such that the local recurrence of cancer and the formation of new blood vessels at the site is inhibited. Within one embodiment of the invention, the anti-angiogenic compound is administered directly to the tumor excision site (e.g., applied by swabbing, brushing or otherwise coating the resection margins of the tumor with the anti-angiogenic compound). Alternatively, the anti-angiogenic compounds may be incorporated into known surgical pastes prior to administration. Within particular embodiments of the invention, the anti-angiogenic compounds are applied after hepatic resections for malignancy, and after neurosurgical operations.

Within one aspect of the present invention, FSAP proteins of the invention and/or polynucleotides encoding FSAP proteins of the invention may be administered to the resection margin of a wide variety of tumors, including for example, breast, colon, brain and hepatic tumors. For example, within one embodiment of the invention, anti-angiogenic compounds may be administered to the site of a neurological tumor subsequent to excision, such that the formation of new blood vessels at the site are inhibited.

The FSAP proteins of the invention and/or polynucleotides encoding FSAP proteins of the invention may also be administered along with other anti-angiogenic factors, such as those described in U.S. Provisional Application Serial No. 60/355,547 and WO 01/79480.

The invention also relates to methods of targeting an antiangiogenic peptide to the inside of a cell or at cell structures in a mammal including humans; methods of targeting the antiangiogenic peptide of the invention to a cell type, target organ, or a specific cytological or anatomical location; methods of diagnosing an anti-angiogenesis related disease or disorder in a mammal including humans; and methods of improving the scheduling of dosing of an antiangiogenic peptide.

Furthermore, chemical entities may be covalently attached to the therapeutic proteins of the invention to enhance or modulate a specific functional or biological activity such as by methods disclosed in Current Opinions in Biotechnology, 10:324 (1999).

Furthermore, tumor or organ targeting entities may be covalently attached to the therapeutic proteins of the invention to target a specific functional or biological activity to certain cell or stage specific types, tissue types or anatomical structures. By directing a therapeutic protein of the invention the action of the agent may be localised. Further, such targeting may enable the dosage of the therapeutic proteins of the invention required to be reduced since, by accumulating the therapeutic proteins of the invention at the required site, a higher localised concentration may be achieved. Therapeutic proteins of the invention can be conjugated with a targeting portion by use of cross-linking agents as well as by recombinant DNA techniques whereby the nucleotide sequence encoding the therapeutic proteins of the invention, or a functional portion of it, is cloned adjacent to the nucleotide sequence of the ligand when the ligand is a protein, and the conjugate expressed as a fusion protein. The targeting agent can be any tumor antigen or organ specific monoclonal antibody, or active portion thereof, eg Fab or F(ab')₂ fragment, a ligand (natural or synthetic) recognised by an endothelial cell surface receptor (e.g. a receptor binding protein) or a functional portion thereof, or any other agent which interacts with protein or structures of the endothelial cell.

In another embodiment of the invention, mutants, variants or fragments of FSAP may be of particular interest, which were modified such that the procoagulant (to activate coagulation factor VII) and/or profibrinolytic (to activate single chain plasminogen activators) enzymatic properties, which in the treatment of angiogenesis-related disorders may be undesired activities, were deleted. A mutant that impairs the profibrinolytic activity of FSAP has been described in DE 100 52 319.6. Deletion of such unwanted enzymatic functions could be done by standard methods known to those skilled in the art, e.g. site directed mutagenesis to alter nucleotide or protein sequences involved, deletion of functional areas, insertion of foreign sequences into functional areas to interrupt the sequence containing the unwanted activity, use of fragments which do not contain the undesired enzymatic functions.

In such case that FSAP fragments were to be considered, which due to their size would be renally cleared rapidly *in vivo*, fusion to carrier molecules such as human serum albumin, antibody constant fragments or transferrin would stabilize such proteins *in vitro* and *in vivo*. The present invention therefore encompasses fusion proteins of an anti-angiogenic FSAP fragment with a protein (e.g., a polypeptide, antibody, or peptide, or fragments and variants thereof), that is sufficient to prolong the shelf life of the protein, and/or stabilize the protein and/or its activity in solution (or in a pharmaceutical composition) *in vitro* and/or *in vivo*. Furthermore, chemical entities may be covalently attached to these fusion protein to enhance or to modulate a biological activity.

The invention also encompasses such fusion proteins which in addition to the carrier molecule and the anti-angiogenically active FSAP fragment contain other polypeptide sequences with known anti-angiogenic properties, e.g. Angiostatin, Endostatin or plasminogen kringle-5. This kind of fusion protein of the invention may have the following formula: R1-C-R2; R2-C-R1; R1-R2-C; R2-R1-C; C-R1-R2; C-R2-R1, wherein R1 is an FSAP derived protein, peptide or polypeptide sequence (including fragments or variants thereof) with anti-angiogenic property, C is a carrier molecule and R2 is another FSAP derived anti-angiogenic protein, peptide or polypeptide sequence (including fragments or variants thereof) or a different anti-angiogenic protein, peptide or polypeptide sequence as stated above. The proteins, polypeptides or peptides may optionally be connected by linkers. Exemplary linkers include (GGGGS)_{N} or (GGGS)_{N} or (GGS)_{N}, wherein N is an integer greater than or equal to 1 and wherein G represents glycine and S represents serine.

The invention also encompasses combination therapy , i.e. any therapy using an anti-angiogenically active FSAP molecule of the invention in addition to other therapy forms, including but not limited to chemotherapy, radiotherapy, immunotherapy, gene therapy, vakzination and other anti-angiogenic substances, in non-advanced and advanced disease states.

Since application of exogeneous FSAP leads to a reduction in angiogenesis, as a corollary, inhibition of endogenous FSAP can increase the level of angiogenesis in a therapeutic setting. The therapeutic application of an angiogenesis-promoting substance, FSAP inhibitors belong to this category, has been extensively discussed by Carmeliet, (Nature Medicine, 2003, 9, pp653-659, Table 2), Substances that inhibit the anti-angiogenic effects of FSAP, and thus promote endogenous angiogenesis, include peptides, proteins, antibodies, nucleotides and chemical substances which interact with either FSAP or its interaction partners or inhibit the protease domain of FSAP.

Having generally described the invention, the same will be more readily understood by reference to the following examples, which are provided by way of illustration and are not intended as limiting. Without further description, it is believed that one of ordinary skill in the art can, using the preceding description and the following illustrative examples, make and utilize the alterations detected in the present invention and practice the claimed methods. The following working examples therefore, specifically point out certain embodiments of the present invention, and are not to be construed as limiting in any way the remainder of the disclosure.

In summary, the examples demonstrate anti-angiogenic and anti-tumor properties of activated FSAP in *in vitro* und *in vivo* experiments and suggest the capability of FSAP to be beneficially used in treating disorders where angiogenesis is involved.

FSAP is a protein with the ability to inhibit the proliferation, DNA synthesis and migration of microvascular endothelial cells. The inhibition of these primary events is evidence for an anti-angiogenic activity in FSAP. In a mouse model of angiogenesis *in vivo* the growth factor-stimulated angiogeneis in matrigel plugs implanted under mouse skin could also be inhibited by the local application of FSAP.

Furthermore in a xenograft mouse tumor model with the human primary pancreatic cancer (BxPC-3), FSAP administered at 8 mg/kg/day s.c. led to a 50% inhibition of tumor growth in SCID mice, demonstrating an anti-tumor growth effect of activated FSAP *in vivo.*

### Examples

For the experiments described in the following examples FSAP was isolated from plasma as previously described, for instance in Example 1 of German patent application DE 199 37 218.7and used either as a proenzyme (single chain FSAP, scFSAP) or after activation (two chain FSAP, tcFSAP).

### Example 1: FSAP inhibits the proliferation of bovine retinal endothelial cells (BREC):

Proliferation of endothelial cells is an inherent step in the process of angiogenesis. The inhibitory activity of FSAP on microvascular endothelial cells was analyzed by a standard proliferation assay involving the counting of cells using an electronic cell-counter. BREC (10,000 cells per well) were plated at day 0 on gelatin-coated 24-well plates in endothelial cell basal medium (ECBM; Promocell, Heidelberg, Germany) with 10% (vol/vol) fetal calf serum (FCS). The next day the cells were washed and resuspended in medium with 0.1% (vol/vol) FCS together with 20 ng/ml vascular endothelial growth factor (VEGF)(R&D Systems, Wiesbaden, Germany) as well as scFSAP (12 µg/ml) or vehicle control (0.2 M Arginine, 0.2 M Lysine, 20 mM NaCl, 5mM Citrate, pH 4.5). ScFSAP and vehicle were added in a final dilution of 1:100 (vol/vol). At day 3 the wells were washed and treated with trypsin to remove the cells and the cell number determined using an electronic cell counter (Schärfe Sytems, Reutlingen, Germany). Cell number per well is shown for different treatment conditions (mean ± SEM). This result indicates that in the presence of FSAP the proliferation of BREC induced by VEGF is completely stopped.

### Example 2: FSAP inhibits DNA synthesis in bovine retinal endothelial cells (BREC):

Cell proliferation is preceded by the duplication of DNA in the S-phase of the cell cycle and the subsequent separation of the daughter nuclei. The inhibitory activity of FSAP was analyzed in microvascular endothelial cells by a standard DNA synthesis assay involving the measurement of the incorporation of 5-bromo-2'-deoxy uridine (BrdU) into the replicating DNA using a kit from Roche Diagnostics GmbH (Mannheim, Germany). BREC (5,000 cells/well) were plated on day 0 in gelatin-coated 96-well plates in ECBM medium with 10% (vol/vol) fetal calf serum (FCS). On day 3 the cells were washed and resuspended in medium with 0.1% (vol/vol) FCS for a further 48 h to reduce the extent of autonomous DNA synthesis. After this "serum-starvation" period fresh 0.1 % (vol/vol) FCS was added the cells were stimulated with VEGF and basic fibroblast growth factor (bFGF) (R& D Systems) (both at 20 ng/ml) as well as scFSAP (12 µg/ml) or vehicle control for a further 20 h. Thereafter BrdU was added to the cells for further 4 h after which the cells were fixed with acidified ethanol. The BrdU incorporated into the DNA was detected with a specific antibody according to the manufacturers instructions. DNA synthesis is shown under different treatment conditions (mean ± SEM) as an absorbance value. The data show that synthesis of DNA associated with the replication of BREC in the presence of VEGF and bFGF is inhibited in the presence of FSAP.

### Example 3: FSAP inhibits migration of Bovine retinal endothelial cells (BREC):

Migration of endothelial cells is an inherent step in the process of angiogenesis. The migration of endothelial cells in the direction of a proangiogenic stimulus precedes the formation of new capillaries. The inhibitory activity of FSAP was analyzed in microvascular endothelial cells by a standard cell migration assay using a modified Boyden chamber (Neuroprobe, Gaithersburg, MD). Polycarbonate filters (Neuroprobe) with 8 µm pores were coated with fibronectin (10 µg/ml) and sandwiched between 2 plastic plates with complementary compartments on the lower and the upper sides. BREC (50,000 cells) were incubated on the upper side of the membrane and the chemotactic agent on the lower side of the membrane. Cells migrate through the polycarbonate filter from the upper to the lower side. ScFSAP (12 µg/ml) or vehicle was added to the cells on the upper side of the membrane in ECBM medium with 0.1% (vol/vol) FCS. After 4 h the membrane was washed and the cells from the upper side (non-migrated cells) were scraped away with a cell scraper. The cells on the lower side (the migrated cells) were fixed with methanol:acetone (1:1) and stained with crystal violet. A densiometric analysis of the membranes was performed and the cell migration is shown under different treatment conditions (mean ± SEM). The data show that the migration of BREC in the direction of a chemotactic gradient of VEGF is diminished in the presence of FSAP.

### Example 4: FSAP inhibits migration of human umbilical vein endothelial cells (HUVEC)

Human umbilical vein endothelial cells, passage 4-8, were maintained in endothelial cell growth (ECG) medium (Promocell, Heidelberg, Germany), 2% fetal bovine serum, 50 ng/ml Amphotericin B and 50 µg/ml Gentamycin, 1 ng/ml bFGF, 0.4% heparin, 0.1 ng/ml EGF, 1 µg/ml Hydrocortisone. Cells were trypsinized, centrifuged and diluted in ECG medium (Promocell, Heidelberg, Germany) with 0.05 % gelatin.
50,000 cells in 250 µL were added per well to 10 mm tissue culture inserts (Nunc, 8 µm pore) that had been treated with 5 µg/ml of fibronectin. Additionally, 50 µl of test compound in different concentrations were added to the insert (resulting volume in the upper part of the insert: 300 µl). Cells were pre-incubated for 20 minutes with or without human scFSAP at a concentration between 200ng - 40µg/ml at 37° C.

Medium (300 µL) was added to the bottom wells with or without 3 ng/ml of VEGF (R&D-Systems, Germany) diluted in ECG medium with 0.05% gelatin and cells were incubated for 6 hours at 37° C. Cells were washed once with PBS and the cells that had not migrated were removed from the top membrane by scraping with a cotton swab. Cells that had migrated were quantitated using a colorimetric assay as follows: cells bound to the bottom of the tissue culture inserts were incubated for 1.5 hours in 400 µL of acid phosphatase substrate (10 mM *p*-nitrophenol phosphate, 10 mM sodium acetate, 0.1 % Triton X-100, pH 5.8) at 37° C. Reaction was then quenched with 100 µL of 1 N NaOH and absorbance of the solution was read at 405 nm. Data (n=3) were calculated as %-inhibition compared to the difference of negative control (without VEGF) subtracted from positive control (with VEGF).

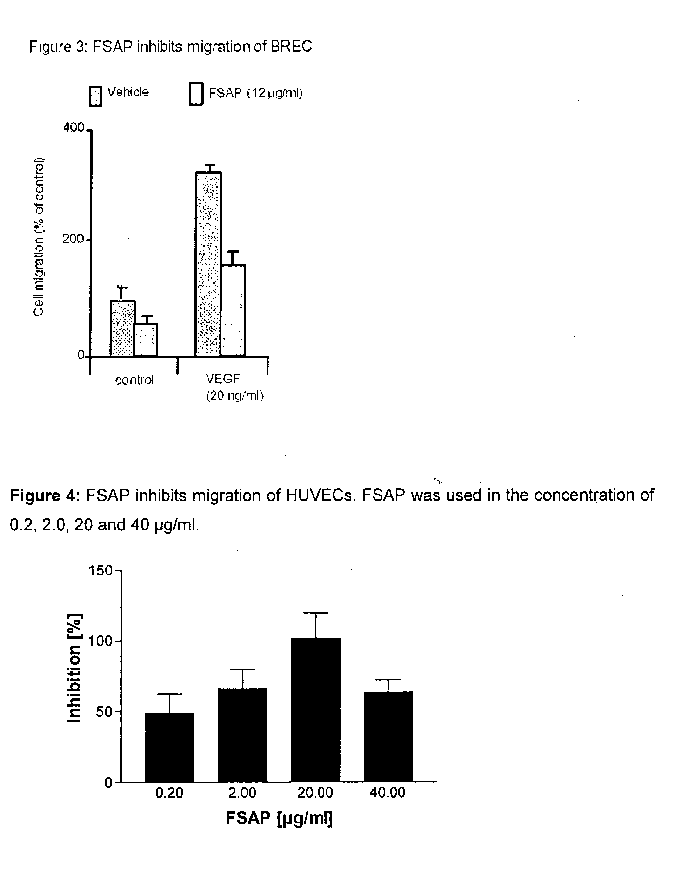

FSAP shows significant dose-dependent efficacy in the migration assay. A dose-dependent inhibition of the VEGF stimulated migration assay with a maximum at 20 µg/ml FSAP (100% inhibition) could be demonstrated (see figure 4). However, lower and higher concentrations of FSAP (e.g. 40 µg/ml) led to a loss of inhibitory activity (see figure 4), indicating a bell-shaped efficacy curve for FSAP *in vitro.*

### Example 5: FSAP inhibits angiogenesis in matrigel plugs implanted in mice:

The development of new capillary network can be investigated *in vivo* by implanting matrigel plugs subcutaneously in mice. Matrigel is an extract of Englebreth-Holm-Swarm sarcoma and consists of extracellular matrix and some growth factors. It exists in the liquid state at 4°C and becomes solid at 37°C. Matrigel (500 µl) (R& D Systems) together with 20 U/ml heparin with or without VEGF (200 ng/ml) and bFGF (200 ng/ml) as well as either scFSAP or tcFSAP (24 µ/ml) or vehicle were injected subcutaneously into the dorso-ventral region of a C57/BL6 mouse; Vehicle A (0.5 mol/I NaCl, 5 mmol/l citrate, pH 4.5) was the carrier for tcFSAP; Vehicle B (0.2 mol/l arginine, 0.2 mol/l lysine, 20 mmol/I NaCl, 5 mmol/l citrate) was the carrier for scFSAP.

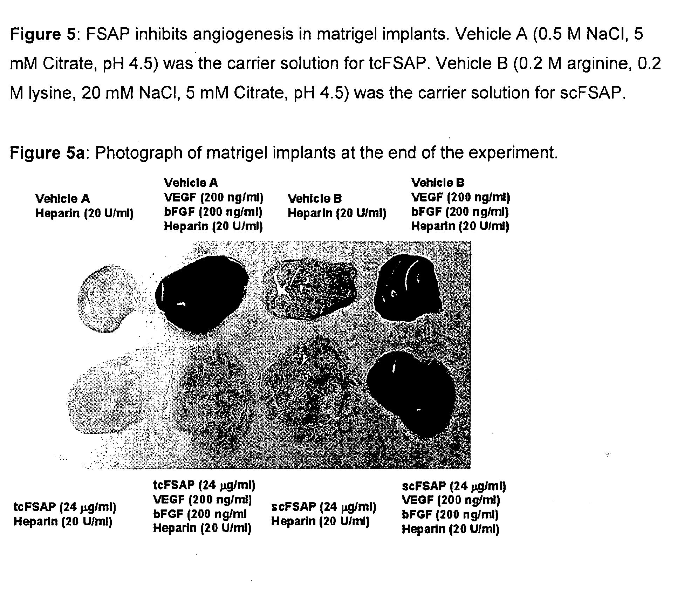

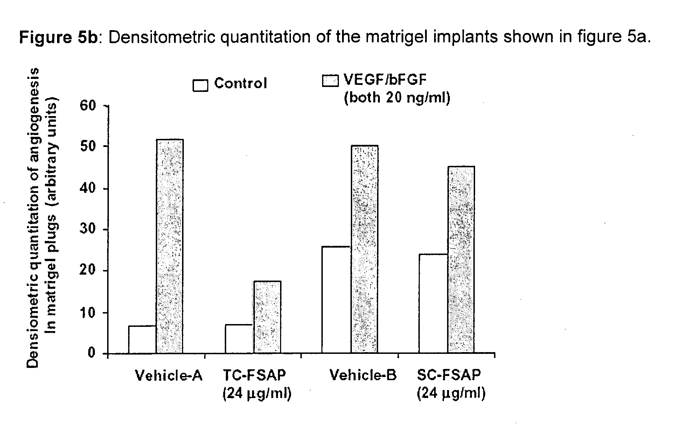

On day 4 the mouse skin was dissected at the site of injection to remove the matrigel plug to analyze the extent of angiogenesis. Incorporation of growth factors (VEGF and bFGF) in the matrigel plug lead to a stimulation of angiogenesis and the number of capillaries in the matrigel plug increase resulting in an entrapment of blood in the matrigel plug which imparts the matrigel a red to orange color (figure 5a). In unstimulated matrigel plugs there were only a few scattered cells and the color remains opaque to white to yellow. Incorporation of FSAP into the matrigel plug, in the presence of VEGF and bFGF, reduced the development of angiogenesis as evidenced by a lack of development of red to orange color (figure 5a). This provides qualitative evidence that FSAP inhibits angiogenesis *in vivo.*

### Example 6: Tumor model

### Cell Culture

Human Pancreatic cancers cells, BxPC-3 (American Type Culture Collection Rockville, MD) were grown in RPMI 1640 with L-Glutamine (Gibco Invitrogen Grand Island, NY) supplemented with 10% fetal calf serum (FCS, Gibco Invitrogen Grand Island, NY) and 1% penicillin/streptomycin (10,000 U/10,000 µg/ml; Biochrom AG, Germany). Cells were maintained in T-75 tissue culture flasks (Cellstar; Greiner Bio-one, Germany) and grown in 5% CO₂ / 95% air at 37° C in a humidified incubator. Cells were washed with PBS, dispersed in a 0.05% solution of trypsin/EDTA (Biochrom AG, Germany), and resuspended. After centrifugation (4000 rpm for 10 min. at room temperature), the cell pellet was resuspended in RPMI 1640 and the concentration was adjusted to 25 x 10⁶ cells/ml.

### Tumor cell implantation and treatment

All animal work was performed in the animal facility at University hospital Marburg in accordance with federal, local, and institutional guidelines. Male SCID mice (severe combined immunodeficiency disease) (Taconic M+B; Denmark), 6-8 weeks old were used. They were acclimated, caged in groups of five in a barrier care facility and fed with animal chow and water *ad libitum.* Animals were anesthetized via inhalation of isofluorane (Abbott, Wiesbaden, Germany) before all surgical procedures and observed until fully recovered. Before tumor cell injection, mice were shaved and the dorsal skin was cleaned with ethanol. A tumor cell suspension (BxPC-3) of 4-5 x10⁶ cells in 0.2 ml RPMI 1640 was injected into the subcutaneous dorsa of mice in the proximal midline. The mice were weighed and tumors were measured every third - fifth day in two diameters with a dial-caliper. Volumes were determined using the formula a² x b x 0.52 (a = longest, b = shortest diameter). At the end of each experiment the mice where killed in accordance with institutional guidelines and the removed tumors were fixed in 10% neutral buffered formalin (Merck, Darmstadt Germany).

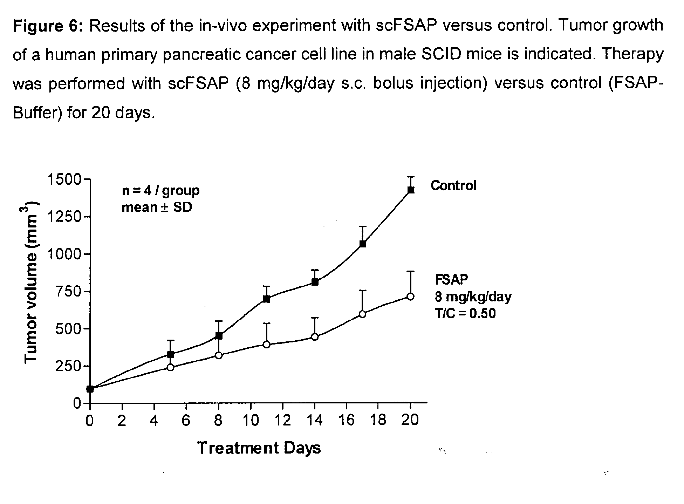

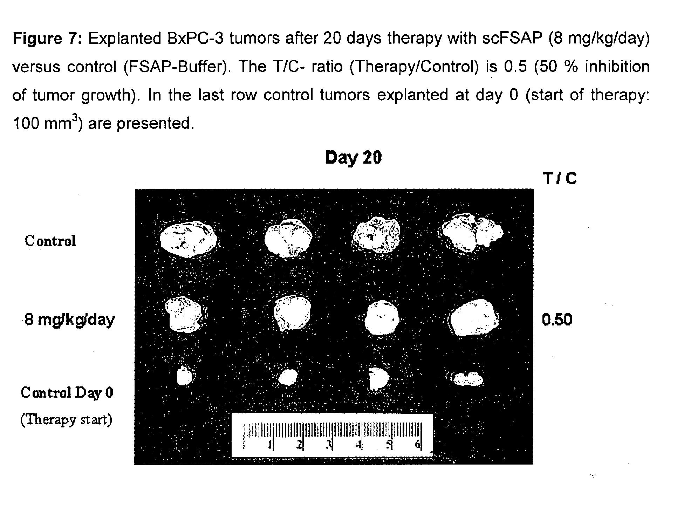

When the tumor volumes were approximately 100 mm³ (within 12-14 days for BxPC-3), mice were randomized into 2 groups (n=4/group). An experiment with scFSAP (8 mg/kg/day) versus FSAP-Buffer (control) was performed. The injections were administered subcutaneously at a site distant from the tumor. Tumors were measured every third to fifth day and the ratio of treated to control tumor volume (T/C) was determined for the last time point. The BxPC-3 tumors (n = 4) were treated for 20 days. In this xenograft mouse tumor model with the human primary pancreatic cancer (BxPC-3), scFSAP (8 mg/kg/day s.c.) led to a 50% inhibition of tumor growth in SCID mice, demonstrating an anti-tumor growth effect of FSAP *in vivo* (figure 6; figure 7, table 1).

**Table 1:**

| Tumor growth of a human primary pancreatic cancer cell line in male SCID mice. Tumor volume (mean ± SD) at each measurement day for both groups. Therapy with scFSAP (8 mg/kg/day s.c. bolus injection) versus control (FSAP-Buffer) for 20 days. | | | | |
|---|---|---|---|---|
| **Day** | **Control** | | **FSAP 8mg/kg/day s.c.** | |
| | **Tumor volume mm**^{**3**} | | **Tumor volume mm**^{**3**} | |
| | **Mean** | **SD** | **Mean** | **SD** |
| 0 | 97 | 8 | 97 | 12 |
| 5 | 328 | 93 | 240 | 100 |
| 8 | 454 | 97 | 319 | 112 |
| 11 | 696 | 87 | 392 | 142 |
| 14 | 811 | 77 | 443 | 128 |
| 17 | 1063 | 116 | 595 | 157 |
| 20 | 1422 | 89 | 711 | 167 |

### Abbreviations

- bFGF: basic fibroblast growth factor
- BrdU: 5-bromo-2'-deoxy uridine
- BREC: bovine retinal endothelial cells
- EDTA: ethylene diamine tetraacetic acid
- FCS: fetal calf serum
- FSAP: factor VII activating protease
- HUVEC: human umbilical vein endothelial cells
- PBS: phosphate buffered saline
- scFSAP: single-chain FSAP (proenzyme)
- SCID: severe combined immunodeficiency disease
- SEM: standard error of the mean
- tcFSAP: two-chain FSAP (activated enzyme)
- VEGF: vascular endothelial growth factor

### Legends to figures

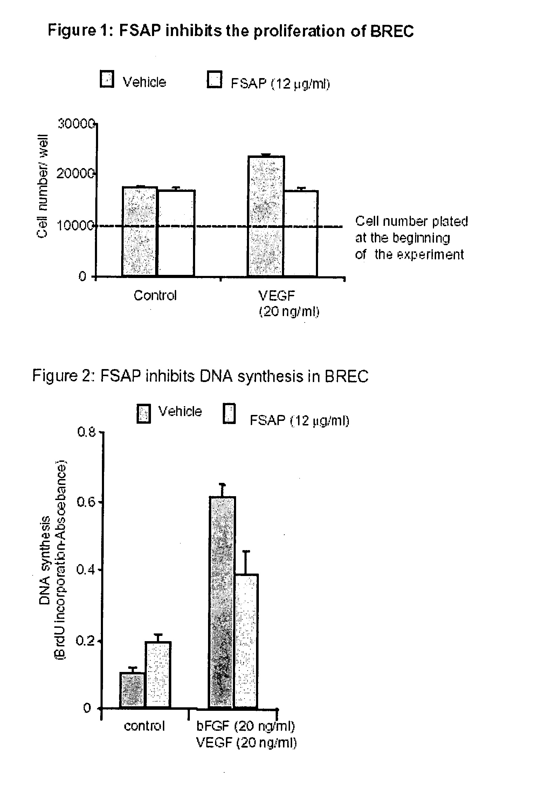

**Figure 1:** FSAP inhibits the proliferation of bovine retinal endothelial cells.
**Figure 2:** FSAP inhibits DNA synthesis in bovine retinal endothelial cells.
**Figure 3:** FSAP inhibits migration of bovine retinal endothelial cells.
**Figure 4:** FSAP inhibits migration of HUVECs. FSAP was used in the concentration of 0.2, 2.0, 20 and 40 µg/ml.
**Figure 5:** FSAP inhibits angiogenesis in matrigel implants. Vehicle A (0.5 M NaCl, 5 mM Citrate, pH 4.5) was the carrier solution for tcFSAP. Vehicle B (0.2 M arginine, 0.2 M lysine, 20 mM NaCl, 5 mM Citrate, pH 4.5) was the carrier solution for scFSAP.
**Figure 5a:** Photograph of matrigel implants at the end of the experiment.
**Figure 5b:** Densitometric quantitation of the matrigel implants shown in figure 5a.
**Figure 6:** Results of the in-vivo experiment with scFSAP versus control. Tumor growth of a human primary pancreatic cancer cell line in male SCID mice is indicated. Therapy was performed with scFSAP (8 mg/kg/day s.c. bolus injection) versus control (FSAP-Buffer) for 20 days.
**Figure 7:** Explanted BxPC-3 tumors after 20 days therapy with scFSAP (8 mg/kg/day) versus control (FSAP-Buffer). The T/C- ratio (Therapy/Control) is 0.5 (50 % inhibition of tumor growth). In the last row control tumors explanted at day 0 (start of therapy: 100 mm³) are presented.

## Claims

1. Factor VII activating protease (FSAP), fragments, mutants and variants thereof which display anti-angiogenic properties.

2. Angiogenesis inhibiting pharmaceutical composition for the prophylaxis, therapy or amelioration of angiogenesis-related disorders containing FSAP according to claim 1.

3. Use of the composition of claim 2 wherein the angiogenesis-related disease is selected from the group consisting of angiogenesis-dependent cancers; benign tumors; rheumatoid arthritis; psoriasis; ocular angiogenesis diseases; Osler-Webber Syndrome; myocardial angiogenesis; plaque neovascularization; telangiectasia; hemophiliac joints; angiofibroma; wound granulation; intestinal adhesions, atherosclerosis, scleroderma, hypertrophic scars, cat scratch disease and *Helicobacter pylori* ulcers.

4. FSAP of claim 1, wherein said proteins or peptides are fused to carrier molecules.

5. FSAP of claim 1 wherein said proteins further comprise a chemical moiety.

6. FSAP of claim 1 wherein said FSAP is modified in such a way that it does not show procoagulant and/or profibrinolytic properties.

7. The composition of claim 2 and a pharmaceutically acceptable carrier or excipient.

8. A method of treating an angiogenesis-related disease or disorder in a patient, comprising the step of administering an effective amount of the composition of claim 2.

9. A method of treating a patient with a solid tumor or hematological cancer that is treatable by angiogenesis inhibiting polypeptides, comprising the step of administering an effective amount of the composition of claim 2.

10. The FSAP of claim 1 further comprising a targeting portion adapted to target the protein to a cell type, target organ, or a specific cytological or anatomical location.

11. A process for providing a mammalian, including a human, cell with FSAP comprising:
introducing a DNA segment encoding for FSAP into a mammalian, including a human, cell, enabling said cell to produce a therapeutically effective amount of FSAP.
